# EUROPÄISCHE PATENTSCHRIFT

(11) **EP 3 518 837 B1**
(45) Veröffentlichungstag und Bekanntmachung des Hinweises auf die Patenterteilung: **16.12.2020**
(21) Anmeldenummer: 17777869.3
(22) Anmeldetag: 27.09.2017
(51) Int. Cl.: A61F 2/915, A61B 17/02, A61B 17/34, A61B 17/00

(54) **RETRAKTOR MIT PUZZLEVERBINDUNG**
RETRACTOR HAVING A PUZZLE-TYPE CONNECTION
ÉCARTEUR À ASSEMBLAGE DE TYPE PUZZLE

(30) Priorität: 30.09.2016 DE 102016118605
(43) Veröffentlichungstag der Anmeldung: 07.08.2019
(73) Patentinhaber: Aesculap AG, 78532 Tuttlingen (DE)
(72) Erfinder: BEGER, Jens, 78532 Tuttlingen (DE); KLINGSEIS, Susanne, 88400 Biberbach (DE); GRIMM, Christian, 78532 Tuttlingen (DE); SERPA, Mario, 78532 Tuttlingen (DE)
(74) Vertreter: Winter, Brandl, Fürniss, Hübner, Röss, Kaiser, Polte - Partnerschaft mbB
(86) Internationale Anmeldenummer: PCT/EP2017/074509
(87) Internationale Veröffentlichungsnummer: WO 2018/060253

(56) Entgegenhaltungen:
- WO-A1-99/37245
- US-A1- 2006 287 706
- US-A1- 2008 195 190
- US-A1- 2010 256 741

## Beschreibung

Die vorliegende Erfindung betrifft einen Stent-Retraktor oder Retraktorstent gemäß dem Patentanspruch 1 und insbesondere Single-use Retraktorstent, dessen segmentale Konstruktion ein individuelles Ablängen (in Axialrichtung) ermöglicht.

### Hintergrund der Erfindung

Ein Retraktor ist allgemein ein chirurgisches Instrument/Bauteil zum Offenhalten bzw. Aufspreizen eines Operationsfeldes/Inzision. Dieses Instrument/Bauteil wird von außerhalb des Patienten in das Operationsfeld eingesetzt und die Spreizelemente voneinander beabstandet. Dadurch wird Binde- und/oder Muskelgewebe (radial) auseinandergedrückt und somit das Operationsfeld erweitert. Die erforderlichen Extensionskräfte werden entweder extrakorporal über ggf. am OP-Tisch montierte Haltearme oder intrakorporal durch Feder- und/oder Stützelemente aufgebracht, welche jeweils die Spreizelemente kraftbeaufschlagen.

### Stand der Technik

Beispielsweise die US 6,187,000 B1 offenbart einen Retraktor dieser Gattung mit einem expandierbaren distalen Ende. Hierbei wird eine Art Folie aus nichtrostendem Metall zu einem Rohr/Trichter zusammengerollt, wobei die aneinanderstoßenden bzw. sich überlappenden Folienkanten miteinander vernietet werden. Eine erste, axial endseitige Niete bildet hierbei ein Schwenkscharnier, wohingegen eine zweite, axialbeabstandete endseitige Niete in einer in der Folie ausgeformten sowie bei zusammengerollter Folie umfangs-umlaufenden Kulisse geführt ist, um so den Durchmesser der Folienrolle abschnittsweise unter Verschwenken um die ersten Niete zu vergrößern oder zu verringern. Dadurch kann eine Zylinder- und eine Trichterform gebildet werden.
Aus der US 8,372,131 B1 ist eine andere Konstruktion für einen Stent-Retraktor der vorliegenden Gattung bekannt. Diese Konstruktion sieht die Anordnung eines Stent-Rohres oder Schlauchs vor, bestehend aus einem zu einem homogenen Drahtgeflecht verarbeiteten Material vorzugsweise mit Memory-Eigenschaften, wobei das Drahtgeflecht innen und/oder Außenseitig mit einer fluiddichten Membran beispielsweise aus PTFE überzogen ist. Der Stent-Schlauch ist zunächst auf ein Dilatationsset montiert, bestehend aus einem Trokarschaft, der von einem Dilatationsballon umgeben ist und der an seinem distalen Ende eine Art Knochenanker in Form eines einzigen, zentral angeordneten Nagels oder einer Knochenschraube hat.

Zur intrakorporalen Platzierung des bekannten Stent-Retraktors wird der Trokarschaft in den Patientenkörper eingeführt und mittels des Nagels oder der Schraube an einem Patientenknochen (z.B. Wirbelknochen) verankert. Anschließend wird der Dilatationsballon aufgepumpt, wodurch sich der Stent-Retraktor radial aufweitet und dabei das umgebende Patientengewebe radial gleichmäßig auseinander drückt. Nach Lösen des Knochenankers und Zurückziehen des Trokarschafts unter Zurücklassen des aufgeweiteten Stent-Retraktors entsteht ein Patientenzugang mit einem Zugangsdurchmesser zur Durchführung einer Operation vorzugsweise mit minimalinvasiven Chirurgie-Instrumenten, welche in den vom Stent-Retraktor definierten Kanal einführbar sind.

Aus der WO 2014/022094 A1 ist allgemein eine textile Struktur mit separaten Stützelementen zur Ausbildung einer Retraktionseinrichtung bekannt. Demnach wird ein zu einem Schlauch geformtes Flechtgewebe durch eine Art separates Stützgerüst radial nach Außen gedrückt, um so eine Aufweitkraft auf das umgebende Patientengewebe aufzubringen. Das Stützgerüst hat ferner eine Anzahl von das Flechtgewebe radial nach Außen durchdringenden Stäben, welche sich im Patientengewebe temporär verankern und damit die Struktur im Patientenkörper axial halten.

Schließlich offenbart die US 20100312189 A1 einen Retraktor, bei dem mehrere Falten bzw. Sicken unterschiedlicher Länge (und damit Abschnitte unterschiedlicher radialer Flexibilität) und miteinander verbunden und sich teilweise überdeckend in einer Wandung eines rohrförmigen Elements erzeugt sind, mittels welchen das rohrförmige Element aufdehnbar und kontrahierbar ist.

Bei den genannten Retraktorsystemen hat sich jedoch gezeigt, dass diese insgesamt eine große Anzahl an Komponenten aufweisen und daher relativ teuer in deren Herstellung sind. Sie sind daher als Einweg-Artikel nicht oder nur bedingt verwendbar. Dies hat einen hohen Zeitaufwand und hohe Kosten für die Wiederaufbereitung von Standardretraktorsystemen zur Folge.

Außerdem sind die dem Stand der Technik bekannten Lösungen nicht individuell anpassbar bzw. ablängbar wodurch eine große Anzahl an verschiedenen Komponenten nötig ist, um auf verschiedene Anwendungsfälle vorbereitet zu sein.

Die US 2010/256741 offenbart eine modulare Stent-Anordnung, die mit Stent-Abschnitten versehen ist, die durch eine Kopplungsanordnung miteinander gekoppelt sind, die aus einem ersten und einem zweiten Kopplungselement besteht. Der modulare Charakter der Stent-Anordnung senkt die Herstellungskosten, kann den Austausch nur eines defekten Teils der Anordnung ermöglichen und die Montage eines Stents mit unterschiedlichen Eigenschaften entlang seiner Länge erleichtern, der für eine bestimmte medizinische Anwendung optimiert ist.

Aus der DE 10 2015 100 933 ist ein Stent mit Retraktorfunktion bekannt, mit einem radial flexibel aufweitbaren Mantel, der in Umfangsrichtung in zumindest zwei stoffeinstückig gefertigte Abschnitte unterschiedlicher radialer Flexibilität unterteilt ist und der über mehrere axiale Segmente verfügt, welche über Sollbruchstellen stoffschlüssig verbunden sind und sich abtrennen lassen. Auf diese Weise lässt sich ein solcher Stent-Retraktor in situ flexibel ablängen und anpassen.

Eine solche Lösung, bei der eine Verbindung der axialen Segmente ausschließlich über Sollbruchstellen erfolgt, hat sich jedoch als ungünstig erwiesen, da, um eine ausreichende Stabilität während einer Operation zu erreichen und ein ungewolltes Ausknicken oder Ausbeulen des Stents sicher verhindern zu können, die Sollbruchstellen vergleichsweise massiv ausgelegt werden müssen, was wiederum die zum Trennen notwendigen Kräfte erhöht und teilweise spezielles Werkzeug zum Ablängen des Stents in situ erforderlich macht. Eine zu hohe Krafteinwirkung auf den Stent beim Ablängen in situ kann zudem Traumata an dem Gewebe verursachen, das den Stent umgibt. Außerdem ergeben sich an den Sollbruchstellen ggf. scharfkantige Bruchkanten, die ein Verletzungsrisiko darstellen.

Die nachfolgend beschriebene Erfindung baut auf dem eben beschriebenen Stent mit Retraktorfunktion auf, macht es sich jedoch zur bevorzugten Aufgabe, ein neues Verbindungs- und Trennkonzept zu entwickeln, das bei gleicher Stabilität der Verbindungselemente im Vergleich zu den im Stand der Technik bekannten Lösungen ein einfacheres, vorzugsweise gratfreies, Abtrennen der axialen Segmente in situ ermöglicht und zugleich die Gefahr von Traumata beim Patienten durch ein solches Abtrennen reduziert.

### Kurzbeschreibunq der Erfindung

Angesichts des vorstehend genannten Stands der Technik ist es die Aufgabe der vorliegenden Erfindung, einen gattungsgemäßen Stent-(Retraktor) bereit zu stellen, der als Wegwerf-Artikel (Single-use Konzept) geeignet bzw. konzipiert ist.

Des Weiteren ist es ein bevorzugtes Ziel der vorliegenden Erfindung, ein theoretisch beliebig formbares und anpassbares (rund, oval etc.) Retraktorsystem bereitzustellen, insbesondere für minimal-invasive Zugänge (z. B. lumbale, thorakale und cervicale Wirbelsäulenzugänge, craniale Anwendungen), welches sich zudem vorzugsweise einfach ablängen lässt, insbesondere auch in-situ, nach dem Einsetzen des Retraktors.

Weiter macht es sich die vorliegende Erfindung zur bevorzugten Aufgabe, dass das Ablängen des Retraktors mit vergleichsweise geringem Kraftaufwand durch einfachste Werkzeuge (bspw. Klemmen, Zangen) oder auch von Hand erfolgen kann und dabei dennoch keine Verletzungsgefahr durch Grate oder Schnittkanten entsteht.

Schließlich ist es ein weiteres bevorzugtes Ziel der vorliegenden Erfindung, dass der Stent beim Fertigungsprozess als eine einstückige Verbindung erzeugt wird bzw. aus einem Rohling gefertigt werden kann und keine Montage nötig ist.

Die vorstehend genannten Aufgaben und Ziele werden erfindungsgemäß mit einem Stent-Retraktor gelöst/erreicht, der zumindest die im Patentanspruch 1 genannten technischen Merkmale aufweist. Vorteilhafte Weiterbildungen des Stent-Retraktors sind Gegenstand der Unteransprüche.

Der Kerngedanke der Erfindung besteht demzufolge darin, den Stent-Retraktor in eine Anzahl von Axial-/Längssegmente (d.h. in axialer Richtung voneinander trennbare Segmente) zu teilen, an denen in Axialrichtung vorragende/rückspringende, sowie zumindest in Axialrichtung wirkende Hinterschneidungen stoffeinstückig ausgeformt sind, die nach dem Puzzlesteine-Prinzip ineinanderpassbar sind.

Puzzlesteine sind in der Regel flache, randseitig mit Vor- und Rücksprüngen konturierte Elemente vorzugsweise aus einem Kartonmaterial, die durch Ineinanderstecken der Vor-/Rücksprünge fest miteinander verbunden werden können. Vorzugsweise haben die Puzzlesteine dabei kopfartige Vorsprünge sowie buchtartige (hufeisenförmige) Rücksprünge/Ausnehmungen, die miteinander so zusammenwirken, dass die miteinander verbundenen Puzzlesteine nichtmehr auseinander gerissen werden können.

Dieses Verbindungsprinzip macht sich die Erfindung zunutze, indem an den vorzugsweise Blech-/Blatt-förmigen (dünnwandigen) Stent-/Retraktor-Segmenten entsprechende Konturen an deren Stirnkanten ausgeformt werden, um dadurch quasi dreidimensionale Puzzlesteine zu bilden, deren Vor- und Rücksprünge ineinander passen (ohne stoffeinstückige Verbindung), um die Segmente in Axialrichtung lösbar aneinander zu halten. Sollen die Segmente dann wieder voneinander getrennt werden, müssen die zusammenwirkenden Vor- und Rücksprünge lediglich senkrecht zur Axialrichtung des Stents auseinander gedrückt werden, ohne dass sich hierdurch eine Bruchkante ergibt.

Um ein unabsichtliches Lösen der Segmentverbindung nach dem Puzzlestein-Prinzip zu vermeiden, können die Vor- und Rücksprünge rand- bzw. kantenseitig vorzugsweise gegenläufig abgeschrägt/angefast sein. D.h. die Kanten/Ränder der die Vor- und Rücksprünge bildenen Konturen sind so zueiander angeschrägt, dass ein Verschieben zweier Segmente senkrecht zur Axialrichtung des Stents durch die gegenläufig ausgerichteten und damit wirkenden Abschrägungen zweier radial sich gegenüberliegenden Vorsprung-Rücksprung-Verbindungen blockiert wird. Anders ausgedrückt sind die Ränder zweier radial sich gegenüberliegender Vorsprung-Rücksprung-Verbindungen radial nach innen abgeschrägt (verjüngen sich radial nach innen), wodurch ein radiales Verschieben zweier miteinander gekoppelter Segmente blockiert wird.

Alternativ hierzu kann es aber auch vorgesehen sein, dass zwei radial sich gegenüberliegende Vorsprung-Rücksprung-Verbindungen (diese sind mindestens für das axiale Arretieren zweier benachbarter Segmente erforderlich) nicht exakt 180° einander gegenüberliegen und somit bei einer gegenseitigen Seitwärtsverschiebung der beiden aneinander gekoppelten Segmente verhaken. Schließlich kann es alternativ vorgesehen sein, das zumindest drei Vorsprung-Rücksprung-Verbindungen vorgesehen sind, die im vorzugsweise gleichmäßigen Umfangsabstand an den Segmenten ausgeformt werden.

Konkreter ausgedrückt wird gemäß einem ersten Aspekt der vorliegenden Erfindung demzufolge ein Stent vorgeschlagen, der zur Verwendung als Retraktor angepasst ist. Hierfür hat der Stent eine radial flexibel aufweitbare, rohrförmige Wandstruktur, die in Umfangsrichtung gesehen in wenigstens zwei Abschnitte mit zueinander unterschiedlicher (radialer) Flexibilität unterteilt ist. Die Abschnitte höherer Flexibilität bzw. Aufweitungsabschnitte dienen dazu, den Durchmesser des Stent verändern zu können und geben dem Stent somit die Eigenschaft, beliebig in Radialrichtung deformiert/aufgeweitet werden zu können (z.B. rund, oval, etc.), wohingegen die Abschnitte niedrigerer Flexibilität bzw. Aussteifungsabschnitte die Standfestigkeit des Stent gegen äußere radiale Kräfte zumindest in bestimmte Radialrichtungen erhöhen und somit helfen, dem Stent eine ausreichende Steifigkeit zu bewahren, um Gewebe im auseinander gedrückten Zustand zu halten. Durch die vorzugweise stoffeinstückige Ausgestaltung des Stent (einzelner Stent-Segmente) ist eine einfache und schnelle Herstellungsart, beispielsweise durch Laser- oder Wasserschneiden von Metallblechen möglich. Dies erlaubt eine wirtschaftliche und praktische Umsetzung eines Single-use Konzepts.

Erfindungsgemäß ist der Stent in axialer Richtung in zumindest zwei voneinander trennbare Segmente (Längssegmente) unterteilt, welche jeweils durch zumindest ein Verbindungselement/Verbindungseinheit (Vor-Rücksprung-Verbindung) miteinander gekoppelt sind. Dabei ist zumindest eines dieser Verbindungselemente als eine Art Puzzleverbindung/Verklinkung ausgebildet, welche sich strukturell/konstruktiv dadurch auszeichnet, dass ein erstes axiales Segment zumindest eine Aussparung bzw. einen konkaves Puzzleabschnitt hat, in der ein einliegender Abschnitt/Teilfläche bzw. ein konvexer Puzzleabschnitt eines zweiten, benachbarten axialen Segments einliegt und die beiden korrespondierenden Puzzleabschnitte durch Formschluss in Axial und Umfangsrichtung zueinander gehalten sind, sich jedoch in Radialrichtung voneinander trennen lassen.

Man könnte auch sagen, dass der Verlauf einer Schnittlinie bzw. Trennkannte (Schnittstelle) zwischen zwei benachbarten, miteinander zu verbindenden Stent-Segmenten zumindest eine Hinterschneidungsstelle bildet, durch die die benachbarten Segmente sowohl in Axialrichtung als auch in Umfangsrichtung durch Formschluss miteinander verbunden sind, jedoch durch eine, lokal im Bereich der Hinterschneidungsstelle stattfindende, radiale Relativbewegung der beiden Segmente zueinander, gelöst werden können.

Aufgrund der Krümmung der rohrförmigen Wandstruktur des Stents, kann eine solche Puzzleverbindung, insbesondere wenn zwei oder mehr solcher Verbindungsabschnitte vorhanden sind, eine Selbsthemmung hervorrufen, da die Puzzleverbindungen jeweils lokal nur in Radialrichtung trennbar sind und diese lokalen Radialrichtungen gegeneinander angestellt sein können, wie dies vorstehend bereits beschrieben wurde. Dies kann dazu führen, dass ein abzutrennendes Segment nur durch eine elastische und/oder plastische Verformung des Stents und/oder durch mehrfaches Hin- und Herbewegen der Segmente relativ zueinander lösbar ist. Auf den Stent wirkende Axial- und Torsionskräfte können hingegen durch den von der Puzzleverbindung gebildeten Formschluss voll übertragen werden, was insbesondere für die Stabilität des Stents beim Einführen in ein Operationsfeld und/oder beim Offenhalten einer Operationsstelle wichtig sein kann. Eine solche Puzzleverbindung bildet also unter Ausnutzung der Krümmung des Stent eine formschlüssige Verbindung zwischen zwei Segmenten, welche eine hohe Stabilität der Verbindungsstelle bei vergleichsweise leichter (Ab-)Trennbarkeit bietet, da sich die einzelnen Puzzleverbindungen durch lokale Radialbewegung (Bewegung in eine Vorzugsrichtung) an der Verbindungsstelle einfach trennen lassen. Auf diese Weise lässt sich ein einfaches Trennkonzept zum flexiblen Ablängen des Stents per Hand oder mit einfachsten Werkzeugen realisieren.

Im Gegensatz zu Sollbruchstellen bzw. stoffschlüssigen Verbindungen, welche die gängigste im Stand der Technik bekannte Lösung für abtrennbare Verbindungselemente bei stoffeinstückig hergestellten Bauteilen bilden, sind die Festigkeit und Steifigkeit der Verbindung bei einer erfindungsgemäßen Puzzleverbindung nicht proportional zur Materialstärke. Wenn man bei einer Sollbruchstelle eine höhere Steifigkeit oder Festigkeit erreichen will, muss man den Durchmesser der Sollbruchstelle erhöhen und erhöht damit gleichzeitig auch die Kraft, die zum Trennen der stoffschlüssigen Verbindung aufgebracht werden muss. Ab einer gewissen Materialstärke der Sollbruchstelle kann sogar Schneidewerkzeug zum Trennen der stoffschlüssigen Verbindung erforderlich sein. Eine erfindungsgemäße Puzzleverbindung bietet demgegenüber den Vorteil, dass bei gleichen oder höheren übertragbaren Lasten ein leichteres Abtrennen der so verbundenen Segmente möglich ist, ohne spezielle Trennwerkzeuge. Zudem besteht bei stoffschlüssigen Verbindungen die Gefahr, dass sich beim Abtrennen scharfe Kanten oder Grate bilden, welche sich in situ nicht mehr entgraten lassen und ein Verletzungsrisiko für Patient und Anwender darstellen, was bei einer erfindungsgemäßen Puzzlestruktur nicht der Fall ist.

Gemäß einer bevorzugten Ausführungsform können die Aussparung und der einliegende Abschnitt symmetrisch ausgebildet sein und der einliegende Abschnitt kann sich vorzugsweise proximal verengen bzw. einen Flaschenhals bilden, um einen Formschluss in Axialrichtung zu erzeugen. Man könnte eine derart angepasste Aussparung mitsamt dem korrespondierenden einliegenden Abschnitt auch als Nut-Feder-Verbindungs-artig ausgebildet bezeichnen, da ein solches Verbindungselement z. B. in der Querschnittsform einer klassischen Puzzleverbindung, Schwalbenschwanzfömig, oder besonders bevorzugt in der Art einer T-Nut und Feder Verbindung ausgebildet sein kann.

Gemäß einer weiteren bevorzugten Ausführungsform, können die Schnittkanten entlang zumindest einer Puzzleverbindung radial auf die Rotationsachse gerichtet sein. Der Begriff Schnittkante bezeichnet dabei allgemein die seitlichen Randflächen aller in der rohrförmigen Mantelfläche des Stents gebildeten (Teil-)Flächen. Eine solche Ausrichtung der Schnittkanten auf die Rotationsachse hat zur Folge, dass die Schnittkanten nicht parallel bzw. schräg gegeneinander angestellt sind und kann eine einfachere Fertigung ermöglichen, da z. B. beim Laserstrahlschneiden der Laserstrahl standardmäßig immer auf die Rotationsachse gerichtet sein kann. Vorzugsweise können dabei die Spaltbreiten zwischen den Schnittkanten relativ klein im Vergleich zur Rohrwandstärke gewählt werden, sodass durch den Anstellungswinkel der Schnittkanten zueinander ein Hinterschnitt in Radialrichtung erzeugt werden kann, durch den die Segmente des Stents im Bereich der Puzzleverbindung sich nur noch durch eine relative Radialbewegung trennen lassen, bei der der einliegende Abschnitt der Puzzleverbindung, relativ zu den in Umfangsrichtung benachbarten Teilflächen, radial nach außen geführt wird. Es ist also in anderen Worten bei einer solchen Ausführungsform nur noch eine Trennung in eine Vorzugsrichtung möglich. Eine solche Ausrichtung der Schnittkanten macht ein unbeabsichtigtes Lösen der Puzzleverbindung unwahrscheinlicher. Die Größe des so entstehenden Hinterschnitts lässt sich über die Spaltbreite zwischen den Schnittkanten und die Wandstärke des Stents einstellen. Die Spaltbreiten werden hierbei bevorzugt relativ klein gewählt (ca. 0,01mm bis 0,1mm, bevorzugt ca. 0,05mm) um auch bei vergleichsweise geringen Wandstärken (ca. 0,5mm bis 1,2mm, bevorzugt 0,7mm bis 1mm) einen ausreichenden Hinterschnitt erzeugen zu können, der sich aber noch manuell lösen lässt.

Um eine weitere Erhöhung der Stabilität zu erreichen, können gemäß einer weiteren Ausführungsform bei zumindest einer Puzzleverbindung die Schnittkanten innerhalb einer einzelnen Puzzleverbindung so ausgebildet sein, dass gegenläufige Flächen geschaffen werden, die zum Lösen in entgegengesetzte Richtungen bewegt werden müssen. Bevorzugt können dazu an einem Puzzle jeweils paarweise Teilflächen unterschiedliche Hinterschnitte haben. So kann sich z. B. ein Teil der Schnittkanten entlang der Puzzleverbindung nur durch ein radiales nach innen Führen lösen lassen, während sich die restlichen Schnittkanten nur durch ein radiales nach außen Führen lösen lassen. Eine solche Gestaltung Hemmt ein Lösen der Puzzleverbindung noch weiter, da sich die einzelne Puzzleverbindung nur durch eine elastische oder plastische Verformung der Puzzleelemente, zur Überwindung der durch die Schnittkanten gebildeten radialen Hinterschnitte, voneinander lösen lassen.

Gemäß einer Ausführungsform der Erfindung kann eine Puzzleverbindung auch so ausgebildet sein, dass Teilflächen zweier benachbarter Segmente jeweils in Umfangsrichtung wechselseitig in die Mantelfläche des jeweiligen anderen Segments hineingreifen/hineinragen und auf diese Weise gegenläufige Flächen geschaffen werden, wobei die Schnittkanten aber trotzdem alle auf die Rotationsachse des Stents gerichtet sein können, was eine einfachere Fertigung ermöglicht.

Gemäß einer bevorzugten Ausbildung der Erfindung kann zumindest eine Puzzleverbindung so ausgebildet sein, dass die proximale Verengung des einlegenden Abschnitts durch zwei an den distalen Rändern der Aussparung ausgebildete hakenförmige Elemente erzeugt wird, welche ihrerseits in die Fläche des zweiten Segments, welches den einliegenden Abschnitt aufweist, hineinragen, und somit auch bei Ausrichtung der Schnittkanten auf die Rotationsachse gegenläufige Flächen gemäß der oben beschriebenen Ausführungsform, und damit eine Selbsthemmung, erzeugen.

Gemäß einer weiteren Ausführungsform kann die Puzzleverbindung mit einer stoffschlüssigen Verbindung/Sollbruchstelle kombiniert werden, d.h. die Schnittlinie entlang der Puzzleverbindung kann an mindestens einer Stelle durch einen Steg stoffschlüssig überbrückt werden, um das Verbindungselement noch weiter zu stabilisieren und ein ungewolltes Verkippen der Puzzleverbindung zu verhindern. Dabei kann der Steg (Sollbruchstelle) relativ klein gewählt werden, z.B. mit einem Durchmesser/Stegbreite von 0,01 mm bis 0,1mm bevorzugt ca. 0,05mm, um ein Abbrechen/Abtrennen der Sollbruchstelle zu vereinfachen.

Es sind also verschiedene Ausführungsformen der Puzzleverbindung vorgeschlagen, durch die, je nach Erfordernissen der Anwendung, die Stabilität der Verbindung erhöht werden kann, indem man zu der ursprünglichen Puzzleverbindung, nach Bedarf, gegenläufige Hinterschnitte und/oder Sollbruchstellen hinzufügt.

Gemäß einer weiteren Ausführungsform der Erfindung, kann der Retraktor-Stent an seinem distalen Ende ein in Axialrichtung längeres durchgängiges Segment aufweisen und an seinem proximalen Ende eine Anzahl kürzerer abtrennbarer Segmente aufweisen. Für das distale lange Segment, welches zuerst in die Operationsstelle eingeführt wird, kann aufgrund der axialen Durchgängigkeit eine höhere Festigkeit und/oder Steifigkeit realisiert werden, während diejenigen proximalen Segmente, die nach dem Einsetzen des Stent-Retraktors aus der Operationsstelle überstehen, einfach abtrennbar sind um den Stent-Retraktor auf eine passende Länge zu kürzen bzw. flexibel abzulängen.

Gemäß einer bevorzugten Ausführungsform kann der Stent pro Segment zumindest zwei Verbindungselemente aufweisen, die sich diametral gegenüberliegend oder in Umfangsrichtung gleichmäßig verteilt angeordnet sein können, um einen gleichmäßigen Kraftfluss zu gewährleisten und das Risiko eines ungewollten Ausknickens beim Aufbringen einer axialen Last, was zu einer Verletzung des Patienten und/oder des Anwenders führen könnte, zu minimieren.

Gemäß einer bevorzugten Ausbildung der Erfindung ist es vorgesehen, dass die Aufweitungsabschnitte jeweils aus einer Anzahl von axial beabstandeten, vorzugsweise elastischen oder plastisch verformbaren Dehnungselementen bestehen, welche jeweils vorzugsweise aus einem in Umfangsrichtung sich erstreckenden Ziehharmonika-Draht gebildet sind. Dadurch kann die Wandstruktur des Stent sowohl im Bereich der Aufweitungsabschnitte als auch im Bereich der Versteifungsabschnitte und der Puzzleverbindungen durch Stanzen oder Schneiden, vorzugsweise Laser- oder Wasserschneiden beispielsweise aus einem (geschlossenen) Rohrprofil hergestellt werden, die nachträglich auch noch entgratet sein können. Die Herstellung ist somit einfach und kostengünstig und daher insgesamt für "single-use" Produkte geeignet.

### Figurenbeschreibung

Die Erfindung wird nachstehend anhand bevorzugter Ausführungsbeispiele unter Bezugnahme auf die begleitenden Figuren näher erläutert
Es zeigen
Fig. 1 eine Darstellung eines Stent gemäß einem ersten bevorzugten Ausführungsbeispiel der vorliegenden Erfindung;
Fig. 2 eine Seitenansicht derselben Ausführungsform;
Fig. 3 eine Schnittansicht durch einen Stent gemäß der ersten bevorzugten Ausführungsform;
Fig. 4 eine weitere Schnittansicht durch einen Stent gemäß der ersten bevorzugten Ausführungsform;
Fig. 5 eine Abwicklung einer Wandstruktur gemäß der ersten bevorzugten Ausführungsform;
Fig. 6 ein Detail der Puzzleverbindung gemäß der ersten bevorzugten Ausführungsform;
Fig. 7 eine Abwicklung einer Wandstruktur gemäß einer zweiten bevorzugten Ausführungsform;
Fig. 8 ein Detail der Puzzleverbindung gemäß der zweiten bevorzugten Ausführungsform;
Fig. 9 ein Detail einer Puzzleverbindung gemäß einer dritten Ausführungsform mit gegenläufigen Hinterschnitten;
Fig. 10 eine Schnittansicht durch eine Puzzleverbindung gemäß einer dritten Ausführungsform;
Fig. 11: eine weitere Schnittansicht durch eine Puzzleverbindung gemäß einer dritten Ausführungsform;
Fig. 12: ein Detail einer Puzzleverbindung gemäß einer vierten Ausführungsform mit gegenläufigen Hinterschnitten; und
Fig. 13: ein Detail einer Puzzleverbindung gemäß einer vierten Ausführungsform mit gegenläufigen Hinterschnitten.

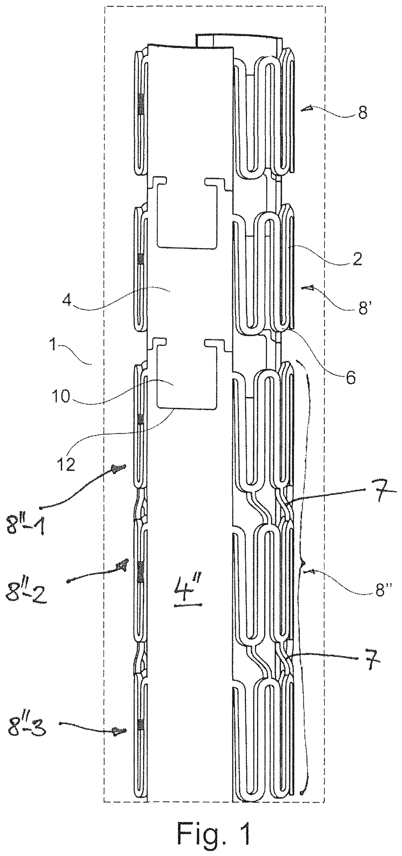

Gemäß der Fign. 1 bis 5 besteht die Wandstruktur eines Stent (-Retraktors) 1 entsprechend einem ersten bevorzugten Ausführungsbeispiel der vorliegenden Erfindung in Umfangsrichtung gesehen aus zwei Umfangsabschnitten mit geringer Steifigkeit 2 (nachfolgend als Aufweitungsabschnitte/-elemente bezeichnet) und zwei Umfangsabschnitten mit vergleichsweise hoher Steifigkeit 4 (nachfolgend als Versteifungsabschnitte/-elemente bezeichnet), die in Umfangsrichtung wechselweise zueinander und in Achsrichtung linear angeordnet sind.

Prinzipiell hat der erfindungsgemäße Stent 1 eine Rohr- oder Schlauchform, wobei die Umfangsabschnitte mit zueinander gleichen/ähnlichen Steifigkeiten einander diametral gegenüberliegen. Der Stent 1 ist ferner stoffeinstückig ausgebildet, d.h. die einzelnen Umfangsabschnitte sind stoffeinstückig miteinander verbunden.

Das vergleichsweise dünnwandige Stentrohr (ca. 0.5 bis 1.5mm) ist vorzugsweise durch Laserstrahlschneiden oder Wasserstrahlschneiden in die genannten Umfangsabschnitte unterteilt. Es sei aber darauf hingewiesen, dass auch andere Bearbeitungstechniken wie Stanzen oder Fräsen zur Herstellung der nachfolgend noch beschriebenen Wandstruktur einsetzbar sind. Je nach Bedarf und Einsatzzweck/-ort kann der Ausgangsdurchmesser (Innendurchmesser des Stentrohrs in Konstruktionslage, d.h. nicht aufgeweitet) in einem Bereich von beispielsweise 10-30mm liegen.

Der Stent 1 hat an seinen Aufweitungsabschnitten 2 im Wesentlichen eine Wandstruktur, die von einem Standard-Gefäßstent (Coroflex) in Grundzügen übernommen ist. Das bedeutet, dass der Stent 1 zumindest im Bereich seiner Aufweitungsabschnitte (Aufweitungselemente) 2 aus einer Anzahl von axial beabstandeten, vorzugsweise parallel verlaufenden Bändern 6 gebildet ist, die sich schlangen- oder ziehharmonikaförmig in Umfangsrichtung erstecken und somit im Bereich ihrer Ziehharmonikaform flexible Aufweitungsreserven in Radialrichtung bilden.

Zur Erhöhung der Stabilität sind die steiferen Umgangsabschnitte (Versteifungsabschnitte) 4 in Umfangsrichtung gesehen jeweils zwischen den beiden Aufweitungsabschnitten 2 (wechselweise) angeordnet. Die Versteifungsabschnitte (Versteifungselemente) 4 werden durch im Wesentlichen geschlossene, vorzugsweise rechteckige Plattenabschnitte ausgebildet, die in ihrer Grundform in Achsrichtung gesehen wannen- oder trogartig gewölbt bzw. rinnenförmig und dafür vorgesehen sind, sich nicht oder nur geringfügig radial aufzuweiten.

Wie ferner aus den Fig. 1 bis 5 zu entnehmen ist, besteht der erfindungsgemäße Stent/Retraktor 1 aus mehreren axial beabstandeten/separaten (Kreis-/Ring-)Segmenten 8, 8', 8", die ebenfalls jeweils aus den vier Umfangsabschnitten bestehen, wie vorstehend beschrieben, wobei die Kreissegmente 8 über die Versteifungsabschnitte 4 durch Verbindungselemente 10 (nicht stoffeinstückig) miteinander verbunden sind. Dieser segmentale Aufbau hat den Vorteil, dass bei einer radialen Aufweitung des Stentdurchmessers dessen Axiallänge im Wesentlichen gleich bleibt, da nur die einzelnen Segmente 8, 8', 8" radial aufgedehnt werden. Ferner können die Verbindungselemente 10 leicht von Hand oder mit einfachsten Werkzeugen, wie z.B. Zangen oder Klemmen abgetrennt werden, sodass eine intra-operative Längenanpassung des Stent 1 möglich ist.

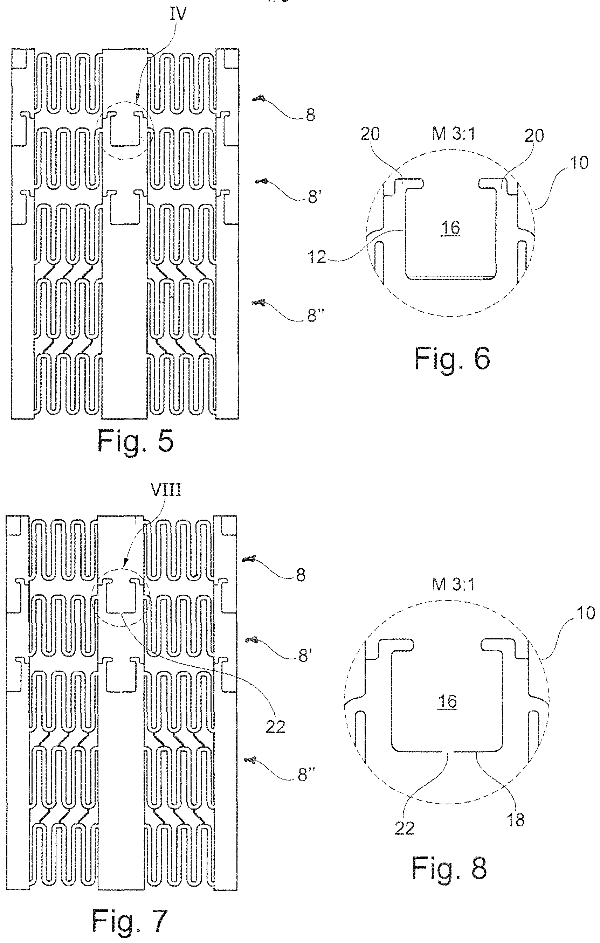

Wie in Fig. 6 dargestellt, werden die Verbindungselemente 10 durch eine an einem axialen Rand (Stirnkante) eines ersten Segments 8' angeordnete Aussparung/Ausnehmung 18 und einen dazu korrespondierenden einliegenden Abschnitt/Vorsprung 16 eines benachbarten zweiten Segments 8 gebildet. In diesem Ausführungsbeispiel ist die Aussparung im Wesentlichen rechteckig ausgebildet und weist an ihren distalen seitlichen Rändern vorzugsweise zwei hakenförmige Teilflächen/Abschnitte 22 auf, die einen Formschluss mit dem einliegenden Abschnitt 16 in axialer Richtung herstellen. Durch diese puzzleverbindungsartig angepassten Verbindungselemente 10, wird ein Formschluss der zwei benachbarten Segmente in Axial- und in Umfangsrichtung hergestellt, während die Verbindung durch eine Bewegung des einliegenden Abschnitts 16 in radialer Richtung lösbar bleibt.

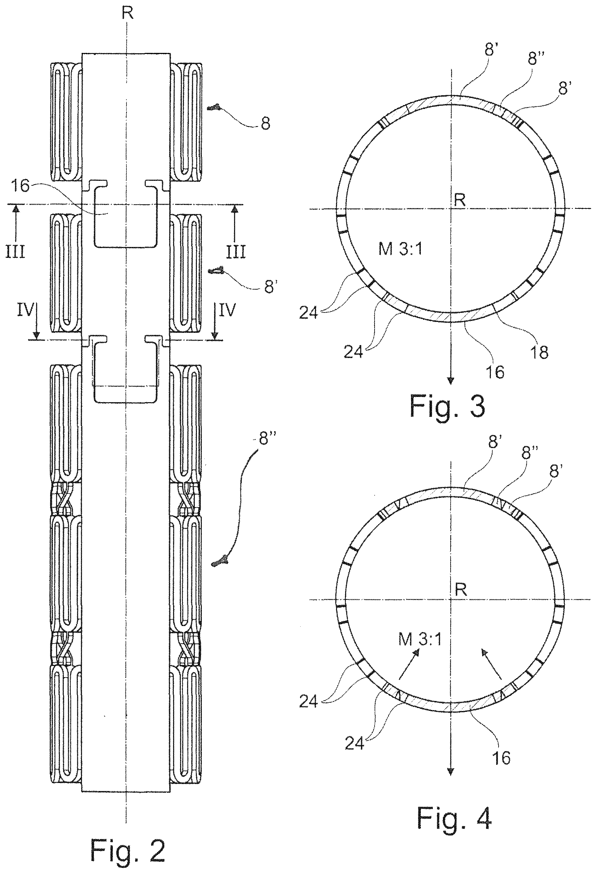

Wie insbesondere die Schnittbilder gemäß der Fign. 3 und 4 zeigen, sind die radialen Schnittkanten im Vorsprungs-/Rücksprungsabschnitt des Stent auf die Rotationsachse R ausgerichtet, bzw. fluchten in dieser. Dies hat zur Folge, dass die in Umfangsrichtung beabstandeten radialen Schnittkanten 24 jedes Vorsprungs-/Rücksprungsabschnitts schräg gegeneinander angestellt sind und, bei passend gewähltem Verhältnis von Spaltbreite zu Wandstärke, radial wirkende Hinterschnitte bilden, sodass sich bestimmte Abschnitte nur in einer bestimmten Vorzugsrichtung (Radialrichtung nach innen) lösen lassen (durch Pfeile angezeichnet). Dadurch, dass die hakenförmigen Teilflächen 22 des ersten Segments 8' in die Fläche des zweiten Segments 8 hineinragen bzw. durch komplementäre hakenförmige Aussparungen im zweiten Segment 8 aufgenommen werden, ergibt sich die Situation, dass sich, um die hakenförmige Teilfläche 22 herum, gegenläufige radiale Hinterschnitte 14, 14' an den Schnittkanten 24 des zweiten Segments 8 bilden (siehe Fig. 4) und dieses deshalb nur durch Überwindung dieser radialen Hinterschnitte 14, 14' durch plastische und/oder elastische Verformung des Materials in diesem Bereich aus der Puzzleverbindung gelöst werden kann, was bei entsprechend vorgesehenen Wandstärken von etwa 0,7 mm bis 1 mm mit geringem Kraftaufwand möglich ist.

Die Fign. 1, 2 und 5 zeigen, dass ein erfindungsgemäßer Stent mit Retraktorfunktion gemäß einer Ausführungsform so aufgebaut sein kann, dass er an seinem distalen Ende, welches als erstes in den Patienten eingeführt wird, ein längeres axiales Segment 8" ausweisen kann, bei dem mehrere schlangenförmige Aufweitungsbänder 6 in den Segmentabschnitten 8"-1, 8"-2 und 8"-3 in Axialrichtung über S-förmige Verbindungsabschnitte 7 stoffschlüssig verbunden und hintereinandergeschaltet in einem Segment 8" als Aufweitungsabschnitt 2 dienen können. Dies verleiht dem distalen Segment 8" in Kombination mit einem durchgängigen Versteifungsabschnitt 4", eine höhere Stabilität gegenüber den kürzeren, abtrennbar über Puzzle-Verbindungselemente 10 angebundenen/gekoppelten Segmenten 8, 8', was sinnvoll sein kann, da der distale Abschnitt beim Einführen in die Operationsstelle höheren Kräften ausgesetzt sein kann und in situ letztlich nur die proximalen Segmente, die aus der Operationsstelle überstehen, abgetrennt werden müssen.

Zur Anwendung kann der erfindungsgemäße Stent mit Retraktorfunktion 1, bspw. Mithilfe eines Trokars in eine Operationsstelle eingeführt werden und nach Entfernen des Trokars einen Zugang zur Operationsstelle offenhalten. Die einzelnen Segmente 8, 8', 8" des Stents 1 können dabei individuell aufgeweitet werden. Nach dem Aufweiten, können proximal überstehende Segmente 8, 8', 8" des Stents 1 mit einfachen Mitteln und geringem Kraftaufwand abgetrennt und der Stent 1 damit auf eine passende Länge gebracht werden.

Gemäß einer weiteren, in den Figuren 7 und 8 gezeigten Ausführungsform, kann ein Verbindungselement 10, zusätzlich zu der oben beschriebenen Puzzleverbindung, eine stoffschlüssige Sollbruchstelle zwischen Aussparung 18 und einliegendem Abschnitt 16 aufweisen, welche quasi steg- oder brückenförmig die Trennlinie 12 zwischen den Abschnitten 8, 8' überbrückt. Auf diese Weise kann eine noch höhere Stabilität der Verbindung erreicht werden, wobei sich die Verbindung, unter Ausnutzung der durch die Puzzleverbindung gebildeten Vorzugsrichtung, jedoch leichter manuell abtrennen lässt, als eine reine stoffschlüssige Verbindung per Sollbruchstelle mit ähnlicher Stabilität. Die Sollbruchstelle kann z.B. mit einem Durchmesser von 0,01 mm bis 0,1mm, bevorzugt ca. 0,05mm, ausgelegt sein.

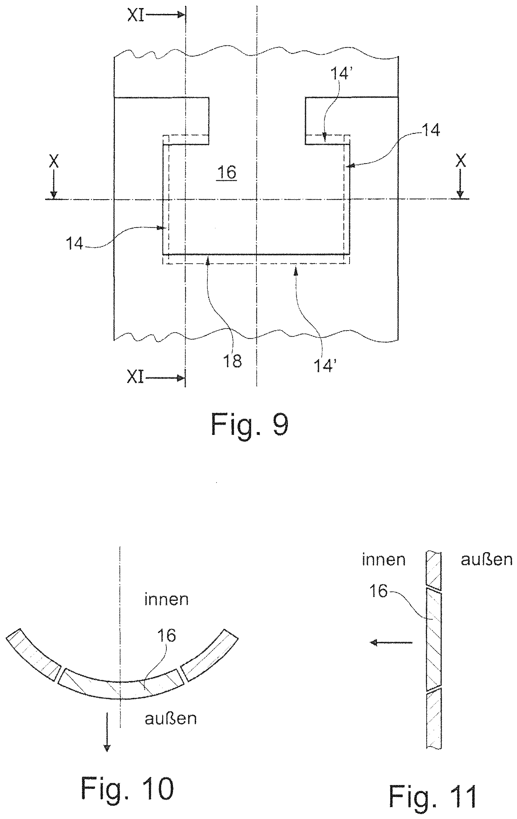

Gemäß weiteren, in den Fign. 9 bis 13 gezeigten Ausführungsformen, können auch durch gezieltes Steuern des Schneidestrahls/Trennwerkzeugs zusätzliche gegenläufige axiale Hinterschnitte 14, 14' an den Schnittkanten erzeugt werden. So können z.B. die axial verlaufenden Schnittkanten 24 entlang einer Puzzleverbindung derart gegeneinander angestellt sein, dass ein einliegender Abschnitt 16 nur radial nach außen lösbar ist (Fig. 10), während die Schnittkanten 24 in Umfangsrichtung derart gegeneinander angestellt sind, dass der einliegende Abschnitt 16 nur radial nach innen lösbar ist (Fig. 11), wodurch die Hinterschnitte 14, 14' sich gegenseitig hemmen und die Verbindung zusätzlich stabilisieren. Der einliegende Abschnitt 16 lässt sich bei einer solchen Ausführungsform also nur durch elastische oder plastische Verformung aus der Puzzleverbindung lösen. Die Schnittbilder zeigen auch gut, dass die radialen Hinterschnitte, durch die gegeneinander angestellten Kanten, im Wesentlichen keilförmige Hinterschnitte bilden.

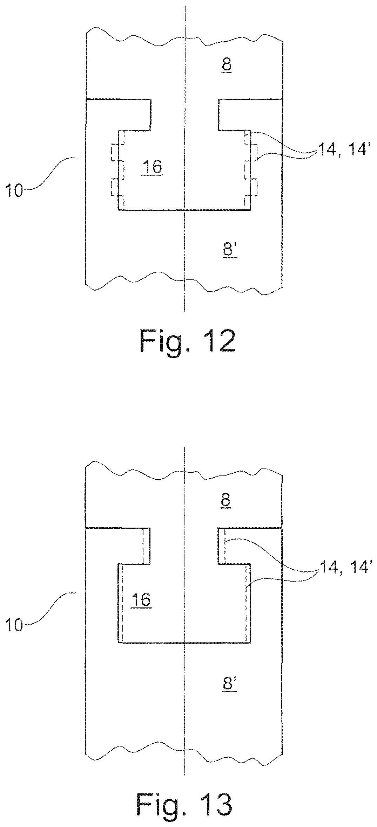

Wie Fig. 12 zeigt, kann die Ausrichtung der radialen Hinterschnitte 14, 14' auch entlang eines einzelnen Schnittkantenabschnitts variiert/alterniert werden um eine Selbsthemmung zu schaffen. Ebenso können auch parallel ausgerichteten Schnittkantenabschnitten entlang der Aussparung 18 sich, vorzugsweise paarweise, gegenseitig hemmen (siehe Fig. 13). Auch jedwede Kombination der oben genannten Schnittkantenausrichtungen sin zur Erzeugung stabilisierender Hinterschnitte denkbar.

Zum Entfernen kann der Stent 1 zerstört werden. Durch einfaches Zusammendrücken kann er auch entsprechend verkleinert und anschließend entnommen werden. Insbesondere für den Fall, dass beim Aufweitvorgang eine (distal) trichterförmige Struktur geschaffen wurde, ist es denkbar, dass beispielsweise durch eine Kompressionszange, die in die Stentstruktur greift, der Stent 1 wieder komprimiert wird.

Als Werkstoff für den erfindungsgemäßen Stent 1 kann Stahl, Titan oder Kunststoff verwendet werden, wobei ein Kunststoffteil vorzugsweise im Spritzgussverfahren hergestellt wird. Des Weiteren kann der Stent 1 nach dem Schneiden der Wandprofile beispielsweise durch Elektropolieren entgratet werden. Um ferner die lichttechnischen Reflektionseigenschaften beispielsweise unter Mikroskopanwendungen zu verbessern, kann zudem die Oberfläche der verbleibenden Strukturen auch mattiert oder beschichtet werden.

Zusammenfassend wird erfindungsgemäß ein Stent vorgeschlagen, der die folgenden Eigenschaften aufweist:
- Single-use Stent(-Retraktor) 1, der beliebig formbar und anpassbar ist;
- segmentale Konstruktion ermöglicht individuelles Ablängen;
- beim Fertigungsprozess wird über Laserschneiden eine einstückige Verbindung hergestellt;
- einzelne Segmente 8, 8', 8" werden durch Puzzleverbindungen unter Ausnutzung der Krümmung des Stents 1 formschlüssig gehalten;
- an den Schnittkanten entlang den Puzzleverbindungen werden radiale Hinterschnitte erzeugt, die ein unbeabsichtigtes Herauslösen der Puzzleverbindungen verhindern; und
- das Puzzleverbindungskonzept kann auch mit Sollbruchstellen kombiniert werden.

Diese Eigenschaften haben die nachfolgenden Vorteile:
- einfaches segmentales Abtrennen des Retraktors, auch in-situ;
- die Länge des Stent 1 muss nicht vor dem Einsetzen bestimmt werden;
- die Segmente können auch nach dem Aufspreizen des Retraktors getrennt werden, da die Hinterschnitte die nötige Festigkeit bieten;
- einfaches Abtrennen bei gleichzeitig hoher Stabilität der Puzzleverbindung;
- Verbindung wird beim Fertigungsprozess erzeugt - es ist keine Montage erforderlich; und
- Zum Trennen können einfache Instrumente, wie Klemmen oder Zangen verwendet werden.

Ausgehend von dem dargestellten Ausführungsbeispiel kann die erfindungsgemäße Haltevorrichtung 1 in vielerlei Hinsicht abgewandelt werden.

So können die Aufweitungsabschnitte eine Vielzahl an Formen annehmen und müssen nicht zwingend Bandförmig ausgebildet sein, sondern können z.B. auch gitter- oder wabenförmig oder als dünnwandiges geschlossenes Blech mit Faltstruktur ausgebilet sein.

Auch ist die Kombination des beschriebenen Stents mit anderen aus dem Stand der Technik bekannten Retraktorkomponenten und -funktionen denkbar, etwa einer Ummantelung, vorzugsweise aus einer Kunststofffolie, zum Gewebeschutz oder Vorrichtungen zum vorübergehenden Verankern des Retraktorstents am Patienten.

### Bezugszeichen

- 1: Stent mit Retraktorfunktion
- 2: Aufweitungsabschnitt
- 4: Versteifungsabschnitte
- 4": durchgängige Versteifungsabschnitt
- 6: Band/ stentförmige Aufweitungsstruktur
- 7: Verbindungsabschnitte
- 8, 8', 8": axiales Segment
- 8"-1: Segmentabschnitt
- 8"-2: Segmentabschnitt
- 8"-3: Segmentabschnitt
- 10: Verbindungselement
- 12: Trennlinie
- 14, 14': radiale Hinterschnitte
- 16: einliegender Abschnitt/konvexe Teilfläche
- 18: Aussparung
- 20: hakenförmige Teilfläche/Verengung
- 22: Steg/Sollbruchstelle
- 24: radiale Schnittkanten
- R: Rotationsachse

## Patentansprüche

1. Stent mit Retraktorfunktion, mit einem radial flexibel aufweitbaren, rohrförmigen Mantel,
welcher in Umfangsrichtung in wenigstens zwei Abschnitte (2, 4) mit unterschiedlicher radialer Flexibilität unterteilt ist, die vorzugsweise stoffeinstückig miteinander verbunden sind, wobei der zumindest eine Abschnitt höherer Flexibilität einen Aufweitungsabschnitt ausbildet und der zumindest eine Abschnitt geringerer Flexibilität einen Versteifungsabschnitt ausbildet und wobei
der Stent in Axialrichtung in eine Anzahl von Segmenten (8, 8', 8") unterteilt ist, die vorzugsweise jeweils interne Versteifungs- und Aufweitungsabschnitte (2, 4) aufweisen und
die Segmente (8, 8', 8") über axiale Verbindungsabschnitte (10) gekoppelt sind, welche Solltrennstellen zur segmentweisen Längenverkürzung bilden,
wobei
die Segmente (8, 8', 8") über die Versteifungsabschnitte (4) durch die Verbindungsabschnitte (10) miteinander verbunden sind und zumindest ein axialer Verbindungsabschnitt (10) dadurch ausgebildet wird, dass ein erstes Segment (8') zumindest eine axial ausgerichtete Aussparung (18) aufweist, in der zumindest ein korrespondierender, einliegender Abschnitt oder axial gerichteter Vorsprung (16) eines zweiten, angrenzenden Segments (8) einliegt, wobei die Aussparung und der einliegende Abschnitt jeweils einen axial wirkenden Hinterschneidungseingriff bilden, durch welche die beiden Segmente (8, 8') in Axial- und in Umfangsrichtung formschlüssig miteinander verbunden sind, jedoch durch eine vorzugsweise lokale, radiale Relativbewegung der Segmente (8, 8') im Bereich des Hinterschneidungseingriffs zueinander, trennbar sind.

2. Stent mit Retraktorfunktion gemäß Anspruch 1, wobei
der einliegende Abschnitt (16) symmetrisch, insbesondere achsensymmetrisch, ausgebildet ist und eine, eine in Axialrichtung wirkende Hinterschneidung ausbildende Verengung/Einschnürung ausbildet und die Aussparung komplementär dazu ausgebildet ist.

3. Stent mit Retraktorfunktion gemäß einem der Ansprüche 1 oder 2, wobei beiden Segmente radiale Schnittkanten (24) aufweisen, die, entlang der Trennlinie (12) zwischen einliegendem Abschnitt (16) und Aussparung (18), radial auf die Rotationsachse des Stents (1) gerichtet und daher derart schräg gegeneinander angestellt sind, dass sie einen radial wirkenden Hinterschnitt bilden, wodurch sich der einliegende Abschnitt (16) nur durch eine lokale Relativbewegung radial nach außen aus der Aussparung (18) lösen lässt.

4. Stent mit Retraktorfunktion gemäß einem der Ansprüche 1 oder 2, wobei die beiden Segmente entlang der Trennlinie (12) zwischen einliegendem Abschnitt (16) und Aussparung (18) Schnittkanten aufweisen, die so angepasst sind, dass sie, insbesondere paarweise, radial gegenläufige Hinterschnitte (14, 14') bilden, die sich nur in entgegengesetzte Richtungen lösen lassen, wodurch der Verbindungsabschnitt (10) nur durch plastische und/oder elastische Verformung in eine Vorzugsrichtung gelöst werden kann.

5. Stent mit Retraktorfunktion gemäß einem der Ansprüche 3 oder 4, wobei die radial gegenläufigen Hinterschnitte (14, 14') durch am ersten Segment (8') ausgebildete hakenförmige Teilflächen (20) an den distalen Rändern der Aussparung (18) erzeugt werden welche in die Mantelfläche des zweiten Segments (8) hineinragen.

6. Stent mit Retraktorfunktion gemäß einem der vorangehenden Ansprüche, wobei zumindest ein Verbindungsabschnitt eine Trennlinie (12) zwischen einliegendem Abschnitt (16) und Aussparung (18) aufweist, wobei die Trennlinie (12) an zumindest einer Stelle stoffschlüssig durch einen Steg (22) überbrückt wird, welcher als zusätzliche Sollbruchstelle dient.

7. Stent mit Retraktorfunktion gemäß einem der vorangehenden Ansprüche, wobei ein am distalen Ende des Stents (1) angeordnetes Segment (8") eine längere Axialerstreckung aufweist, als relativ dazu gesehen proximal angeordnete abtrennbare Segmente (8, 8').

8. Stent mit Retraktorfunktion gemäß einem der vorangehenden Ansprüche, wobei die Spaltbreite(n) der Trennlinie (12) zwischen einliegendem Abschnitt (16) und Aussparung (18) Bereich 0,01 mm bis 0,1 mm, insbesondere ca. 0,05 mm, liegen und die Wandstärke des rohrförmigen Mantels des Stent Bereich 0,5 mm bis 1,2 mm liegt.

9. Stent mit Retraktorfunktion gemäß Anspruch 1, wobei zumindest an einem Segment (8, 8', 8") zumindest zwei Verbindungsabschnitte (10) diametral gegenüberliegend bzw. in Umfangsrichtung gleichmäßig beabstandet, insbesondere in Versteifungsabschnitten (4), ausgebildet sind.

10. Stent mit Retraktorfunktion nach einem der vorstehenden Ansprüche, wobei die Aufweitungsabschnitte (2) und die Versteifungsabschnitte (4) mit den darin angeordneten Strukturen (16) durch Laser- oder Wasserschneiden eines Rohr-Rohlings hergestellt sind.

11. Stent mit Retraktorfunktion nach einem der vorstehenden Ansprüche, wobei der Mantel in wenigstens einen Aufweitungsabschnitt (2) mit höherer radialer Flexibilität und wenigstens einen Versteifungsabschnitt (4) mit geringerer radialer Flexibilität unterteilt ist, welche in Umfangsrichtung wechselweise zueinander und in Axialrichtung linear zueinander angeordnet sind.

## Claims

1. A stent with retractor function, comprising a radially flexibly expandable tubular sheath
which is divided in its peripheral direction into at least two sections (2, 4) of different radial flexibility, which are preferably interconnected integrally, wherein the at least one section with higher flexibility forms an expanding section and the at least one section with lower flexibility forms a reinforcing section, and
the stent being divided in its axial direction into a number of segments (8, 8', 8"), which preferably each have internal reinforcing and expanding sections (2, 4) and
the segments (8, 8', 8") are coupled via axial connecting sections (10) which form nominal separation points for segmental length shortening,
wherein
the segments (8, 8', 8") are connected to each other via the reinforcing sections (4) by the connecting sections (10) and at least one axial connecting section (10) is formed by a first segment (8') having at least one axially oriented recess (18) in which at least one corresponding, inserted section or axially oriented protrusion (16) of a second, adjacent segment (8) is fitted, wherein the recess and the inserted section each forms an axially acting undercut engagement by means of which the two segments (8, 8') are positively connected to one another in axial and peripheral direction, but can be separated by a preferably local, radial relative movement of the segments (8, 8') in the region of the undercut engagement.

2. The stent with retractor function according to claim 1, wherein the inserted section (16) is designed to be symmetrical, in particular axially symmetrical, and forms a constriction/narrowing which defines an undercut acting in the axial direction, the recess being designed to be complementary thereto.

3. The stent with retractor function according to one of claims 1 or 2, wherein the two segments comprise radial cutting edges (24) along the separation line (12) between the inserted section (16) and the recess (18), which are directed radially onto the axis of rotation of the stent (1) and are therefore set obliquely relative to each other so as to form a radially acting undercut, whereby the inserted section (16) can only be released radially outwards from the recess (18) by a local relative movement.

4. The stent with retractor function according to one of claims 1 or 2, wherein the two segments comprise cutting edges between the inserted section (16) and the recess (18) along the separation line (12) which are adapted so as to form, in particular in pairs, radially counteracting undercuts (14, 14') which can only be released in opposite directions, whereby the connecting section (10) can only be released by a plastic and/or elastic deformation occurring in a preferred direction.

5. The stent with retractor function according to one of claims 3 or 4, wherein the radially counteracting undercuts (14, 14') are produced by hook-shaped partial surfaces (20) formed on the first segment (8') at the distal edges of the recess (18), which project into the lateral surface of the second segment (8).

6. The stent with retractor function according to one of the preceding claims, wherein at least one connection section (12) comprises a separation line (12) between the inserted section (16) and the recess (18), wherein the separation line (12) is bridged by a web (22) at least one at point in an integrally bonded manner, wherein the web serves as an additional predetermined breaking point.

7. The stent with retractor function according to one of the preceding claims, wherein a segment (8") arranged at the distal end of the stent (1) has a longer axial extension than separable segments (8, 8') arranged proximally thereto.

8. The stent with retractor function according to one of the preceding claims, wherein the gap width(s) of the separation line (12) between the inserted section (16) and the recess (18) lie in the range from 0.01 mm to 0.1 mm, in particular amount to approx. 0.05 mm, and the wall thickness of the tubular sheath of the stent (1) ranges from 0.5 mm to 1.2 mm.

9. The stent with retractor function according to claim 1, wherein at least two connecting sections (10) are formed on at least one segment (8, 8', 8") diametrically opposed or evenly spaced in the peripheral direction, in particular in reinforcing sections (4).

10. The stent with retractor function according to one of the preceding claims, wherein the expanding sections (2) and the reinforcing sections (4) complete with the structures (16) arranged therein are produced by laser or water jet cutting of a tubular blank.

11. The stent with retractor function according to one of the preceding claims, wherein the sheath is divided into at least one expanding section (2) with higher radial flexibility and at least one reinforcing section (4) with lower radial flexibility, which are arranged alternately relative to one another in the peripheral direction and linear relative to one another in the axial direction.

## Revendications

1. Stent muni d'une fonction d'écarteur, avec une gaine tubulaire radialement flexible et pouvant être écartée,
lequel est divisé, dans la direction circonférentielle, en au moins deux sections (2, 4) avec une flexibilité radiale différente, qui de préférence sont reliées entre elles en une seule pièce, l'au moins une section de plus grande flexibilité formant une section d'écartement et l'au moins une section de plus petite flexibilité formant une section de rigidification et
le stent étant divisé dans la direction axiale en un nombre de segments (8, 8', 8"), qui de préférence présentent respectivement des sections internes de rigidification et d'écartement (2, 4) et
les segments (8, 8', 8") étant couplés par l'intermédiaire de sections de liaison axiales (10), lesquels forment des zones de séparation théorique pour raccourcir la longueur par segment,
les segments (8, 8', 8") étant reliés entre eux par l'intermédiaire des sections de rigidification (4) par les sections de liaison (10) et au moins une section de liaison axiale (10) étant formée de telle sorte qu'un premier segment (8') présente au moins un évidement orienté axialement (18), dans lequel est insérée l'au moins une section insérée correspondante ou une saillie orientée axialement (16) d'un deuxième segment adjacent (8), l'évidement et la section insérée formant respectivement un engagement en contre-dépouille agissant axialement, par lequel les deux segments (8, 8') sont reliés entre eux par complémentarité de forme dans les directions axiale et circonférentielle, toutefois par un mouvement relatif de préférence local et radial les segments (8, 8') peuvent être séparés l'un de l'autre dans la zone d'engagement en contre-dépouille.

2. Stent muni d'une fonction d'écarteur selon la revendication 1, dans lequel la section insérée (16) est formée de manière symétrique, en particulier de manière symétrique par rapport à l'axe, et forme un rétrécissement/étranglement formant une contre-dépouille agissant dans la direction axiale et l'évidement est formé de manière complémentaire à celle-ci.

3. Stent muni d'une fonction d'écarteur selon l'une quelconque des revendications 1 ou 2, dans lequel les deux segments présentent des arêtes de coupe radiales (24), qui sont orientées radialement sur l'axe de rotation du stent (1) le long de la ligne de séparation (12) entre la section insérée (16) et l'évidement (18) et par conséquent sont engagés l'un contre l'autre de manière oblique de sorte qu'ils forment une contre-dépouille agissant radialement, moyennant quoi la section insérée (16) ne peut être détachée de l'évidement (18) que par un mouvement relatif local radialement vers l'extérieur.

4. Stent muni d'une fonction d'écarteur selon l'une quelconque des revendications 1 ou 2, dans lequel les deux segments présentent des arêtes de coupe entre la section insérée (16) et l'évidement (18) le long de la ligne de séparation (12), qui sont adaptées de telle sorte qu'elles, en particulier par paire, forment des contre-dépouilles (14, 14') opposées radialement, qui ne peuvent être détachées que dans des directions opposées, moyennant quoi la section de liaison (10) ne peut être détachée que par déformation plastique et/ou élastique dans une direction préférentielle.

5. Stent muni d'une fonction d'écarteur selon l'une quelconque des revendications 3 ou 4, dans lequel les contre-dépouilles (14, 14') opposées radialement sont produites par des surfaces partielles en forme de crochet formées au niveau du premier segment (8') sur les bords distaux de l'évidement (18), lesquelles font saillies dans la surface de la gaine du deuxième segment (8).

6. Stent muni d'une fonction d'écarteur selon l'une quelconque des revendications précédentes, dans lequel au moins une section de liaison présente une ligne de séparation (12) entre la section insérée (16) et l'évidement (18), la ligne de séparation (12) étant pontée à au moins un endroit par liaison de matière par un pontet (22), lequel sert de point de rupture supplémentaire.

7. Stent muni d'une fonction d'écarteur selon l'une quelconque des revendications précédentes, dans lequel un segment (8") disposé au niveau de l'extrémité distale du stent (1) présente une étendue axiale plus longue, par rapport aux segments (8, 8') détachables disposés de manière proximale.

8. Stent muni d'une fonction d'écarteur selon l'une quelconque des revendications précédentes, dans lequel la(les) largeur(s) de fente de la ligne de séparation (12) se situent entre la section insérée (16) et l'évidement (18) dans la plage allant de 0,01 mm à 0,1 mm, en particulier est d'env. 0,05 mm, et l'épaisseur de paroi de la gaine tubulaire du stent (1) se situe dans la plage allant de 0,5 mm à 1,2 mm.

9. Stent muni d'une fonction d'écarteur selon la revendication 1, dans lequel, au moins au niveau d'un segment (8, 8', 8") au moins deux sections de liaison (10) diamétralement opposées ou équidistantes dans la direction circonférentielle, en particulier sont formées dans des sections de rigidification (4).

10. Stent muni d'une fonction d'écarteur selon l'une quelconque des revendications précédentes, dans lequel les sections d'écartement (2) et les sections de rigidification (4) avec leurs structures (16) disposées dans celles-ci sont fabriquées par découpe au laser ou au jet d'eau d'une préforme tubulaire.

11. Stent muni d'une fonction d'écarteur selon l'une quelconque des revendications précédentes, dans lequel la gaine est divisée en au moins une section d'écartement (2) avec une flexibilité radiale plus grande et au moins une section de rigidification (4) avec une flexibilité radiale plus petite, lesquelles sont disposées alternativement entre elles dans la direction circonférentielle et linéairement entre elles dans la direction axiale.
